# FASCICULE DE BREVET EUROPEEN

(11) **EP 1 014 892 B1**
(45) Date de publication et mention de la délivrance du brevet: **30.07.2003**
(21) Numéro de dépôt: 98900642.4
(22) Date de dépôt: 30.01.1998
(51) Int. Cl.: A61F 2/08, A61B 17/04, A61B 17/11

(54) **DISPOSITIF DE RATTRAPAGE D'UN TENDON ROMPU**
VORRICHTUNG ZUM ERGREIFEN EINER GERISSENEN SEHNE
DEVICE FOR REPAIRING RUPTURED ACHILLES TENDON

(30) Priorité: 05.02.1997 CH 24697
(43) Date de publication de la demande: 05.07.2000
(73) Titulaire: Assal, Mathieu, 1207 Genève (CH)
(72) Inventeur: Assal, Mathieu, 1207 Genève (CH)
(74) Mandataire: Martin, Didier
(86) Numéro de dépôt international: IB9800121
(87) Numéro de publication internationale: WO98034566

(56) Documents cités:
- US-A- 4 723 548
- US-A- 5 251 642
- SOVIET INVENTIONS ILLUSTRATED Section PQ, Week 8629 1 août 1986 Derwent Publications Ltd., London, GB; Class P31, AN 86-188399 XP002060785 & SU 1 169 626 A (DANILOV A A) , 30 juillet 1985
- KAKIUCHI M.: "A COMBINED OPEN AND PERCUTANOUS TECHNIQUE FOR REPAIR OF TENDO ACHILLIS" JOURNAL OF BONE AND JOINT SURGERY. BRITISH VOLUME, vol. 77, no. 1, janvier 1995, UNITED KINGDOM, pages 60-63, XP002060784

## Description

La présente invention est relative à un dispositif utilisé, lors d'une opération chirurgicale de réparation d'un tendon d'Achille rompu, pour rattraper l'extrémité de tendon solidaire du muscle triceps et situé au sein de sa gaine péritendineuse. Ce dispositif peut également être utilisé pour rattraper d'autres tendons du corps humain.

Le tendon d'Achille est susceptible de se rompre suite à un traumatisme accidentel ou suite à une dégénérescence due à l'âge. Usuellement, le tendon se rompt transversalement à environ 4 cm au-dessus de son insertion sur le calcanéum, et remonte de par la contraction du triceps plus ou moins haut à l'intérieur de sa gaine péritendineuse.

L'opération chirurgicale de réparation consiste à rattraper l'extrémité supérieure du tendon pour le tirer vers le bas jusqu'à le ramener bout-à-bout avec l'extrémité inférieure solidaire du calcanéum, et à les suturer ensemble. Cette opération doit donc restaurer la longueur originelle du tendon, minimiser l'adhérence entre le tendon et les tissus l'entourant et laisser un minimum de cicatrice.

Différentes techniques de récupération de tendon plus ou moins invasives sont utilisées selon les centres hospitaliers. Une technique invasive consiste à faire une longue incision, de l'ordre de 15 cm, dans la zone cutanée postérieure de la jambe le long du muscle triceps puis du tendon jusqu'au calcanéum pour aller retrouver les deux extrémités du tendons et les remettre ensemble. Toutefois, cette technique est désavantageuse de par la longueur importante de l'incision du tissu cutané et du péritendon, source de vascularisation du tendon lui-même. De plus, cette zone présente une vascularisation critique et est fortement sollicitée par le frottement avec l'arrière des chaussures.

Une technique moins invasive est décrite dans l'article "a combined open and percutaneous technique for repair of tendo achillis" publié en janvier 1995 dans "the Journal of Bone and Joint Surgery". Selon cette technique, on commence par ne pratiquer qu'une petite incision de 2 à 3 cm longitudinale au niveau de l'extrémité du bout du tendon solidaire du calcanéum. On introduit ensuite deux tiges torsadées relativement rigides dans la gaine péritendineuse pour placer leurs extrémités formant une boucle de part et d'autre de l'extrémité de tendon solidaire du triceps. Une longue aiguille munie d'un fil est alors passée respectivement au travers de la peau adjacente, de la boucle de la première tige, du tendon, de la boucle de la seconde tige avant de traverser et de ressortir de la peau opposée. Lorsque le fil est en place, on ressort les tiges ce qui amène le fil engagé dans le tendon hors de l'incision. Cette procédure est réitérée une ou deux fois à différents niveaux du tendon. On peut alors tirer de manière homogène sur les trois fils pour ramener facilement l'extrémité supérieure du tendon en place et le suturer à l'extrémité inférieure. Si désiré, les fils de traction sont eux mêmes reliés latéralement à d'autres fils engagés en correspondance dans l'extrémité inférieure du tendon.

Toutefois, la difficulté majeure de cette technique réside dans le fait qu'il est difficile de retrouver l'orifice de la boucle supérieure de l'une puis de l'autre tige torsadée lorsque celles-ci sont sous la peau et que l'on désire y introduire l'aiguille.

Lorsque l'on travaille ainsi à l'aveugle, on essaie de reconnaître les positions respectives des boucles par palpation, ce qui est long et fastidieux. De plus, en final, on n'est jamais sûr que l'aiguille soit effectivement passée dans la boucle avant de retirer les tiges hors de la gaine. L'opération peut alors prendre beaucoup de temps car on doit usuellement implanter pas moins de six fils. Ceci est préjudiciable du fait que l'on prolonge la durée de l'anesthésie.

Le but de la présente invention est un dispositif comprenant un instrument et une aiguille adaptée facilitant notablement l'opération chirurgicale de réparation d'un tendon, notamment d'Achille par le procédé sous-cutané peu invasif décrit précédemment. Autant que possible, ce dispositif doit éviter tout risque de blessure additionnelle, être de conception relativement simple pour être facile à mettre en oeuvre et peu coûteux à réaliser. Ce dispositif doit ainsi permettre de réduire sensiblement le temps moyen nécessaire pour une telle opération, tout en permettant de réaliser une parfaite suture du tendon par la suite.

Ces buts sont réalisés grâce un dispositif comprenant un instrument composé d'une paire de branches internes prévues pour être insérées dans la gaine de part et d'autre du tendon et d'une poignée externe de manipulation dont un élément est situé dans le plan de la paire de branches et en vis-à-vis, l'élément et la paire de branches internes présentant respectivement au moins un orifice selon un même alignement passant par le tendon ; et une aiguille susceptible d'être insérée dans l'orifice de l'élément pour être guidée afin de passer par les orifices de branches internes.

Ainsi, après introduction de la paire de branches internes dans la gaine péritendineuse et positionnement de ces branches de part et d'autre du tendon, ce qui est confirmé aisément par palpation, l'orifice de l'élément de poignée permet de guider l'aiguille dans une direction précise lui permettant de passer immanquablement et sans visibilité au travers du tendon qui est de plus retenu par ces branches lors de son percement. L'opération d'enfilage d'un fil dans ce tendon devient alors particulièrement rapide, ce qui diminue d'autant la durée de l'intervention chirurgicale.

De préférence, l'élément de poignée et la paire de branches internes présentent respectivement un même réseau d'orifices alignés, si désiré, les embouchures des orifices des branches étant chanfreinées.

Cette caractéristique permet d'augmenter avantageusement les chances de succès de passage rapide de l'aiguille au travers du tendon, quand bien même la paire de bras n'est pas exactement alignée avec ce tendon. De plus, ce réseau d'orifices permet de passer plusieurs fils avant de retirer l'instrument. Les embouchures chanfreinées des orifices permettent de rattraper facilement de légères déviations de l'aiguille hors de l'alignement des orifices.

De préférence, les deux branches internes forment entre elles un angle d'angle au sommet compris entre 2° et 8°, de préférence 4°, ouvert dans le sens d'introduction. Cette caractéristique permet à la paire de branches de venir épouser le contour conique de l'extrémité de tendon sans risquer de la repousser plus loin en arrière dans sa gaine.

De préférence, les branches internes de l'instrument présentent une section transversale rectangulaire aplatie aux coins arrondis, ou une section ovale aplatie, si désiré la plus grande longueur de cette section se rétrécissant vers l'extrémité. Ces branches internes se glissent alors mieux entre le tendon et sa gaine péritendineuse lors de l'installation de l'instrument.

Avantageusement, l'écartement entre les deux branches internes est réglable. L'instrument peut alors être adapté aux différentes morphologies des patients.

Avantageusement, l'élément de la poignée comprend deux branches externes respectivement situées de part et d'autre de la paire de branches internes dans un même plan. L'instrument peut alors être confortablement utilisé aussi bien par un chirurgien droitier que gaucher lorsqu'il saisit l'instrument par l'une des branches externes.

Alors, l'instrument est de préférence constitué de deux pièces sensiblement en U et assemblées côte-à-côte par un mécanisme permettant de régler l'écartement entre les pièces, les branches de U adjacentes de chacune des pièces formant la paire de branches internes, les branches éloignées formant la poignée. La conception de cet instrument s'avère particulièrement simple et facile à réaliser. Mieux, les deux pièces en U sont symétriques par rapport à un plan orthogonal, et la base de jonction des branches des pièces en U est arquée. L'apparence encore plus simplifiée de l'instrument facilite sa manipulation d'un côté comme de l'autre. La jonction arquée des pièces facilite l'introduction des branches internes dans la gaine sans que l'instrument vienne frotter contre le talon du pied.

Utilement, le mécanisme d'assemblage et de réglage des deux pièces côte-à-côte comprend au moins une tige solidaire de l'une des pièces et coulissant dans un perçage ménagé en correspondance dans l'autre pièce ainsi qu'une vis montée mobile en rotation dans l'une des pièces avec son filetage engagé dans un trou taraudé ménagé dans l'autre pièce, la tête de la vis étant apparente à l'extérieur pour manipulation. Ce mécanisme de réglage est également simple à réaliser et évident à mettre en oeuvre.

De préférence, le chas de l'aiguille présente une épaisseur réduite. La réduction de l'épaisseur du chas tient compte de l'épaisseur du fil, le chas et le fil pouvant passer dans les orifices sans risque de cisaillement, voire de coupe, du fil.

Utilement, l'instrument et l'aiguille sont réalisés en acier inoxydable, ou l'instrument à usage unique est réalisé en plastique.

L'invention sera mieux comprise à l'étude d'un mode de réalisation pris à titre d'exemple nullement limitatif et illustré par les figures suivantes :
- la figure 1 est une vue en perspective du dispositif selon l'invention, et
- la figure 2 est une vue de côté d'une variante de l'instrument.

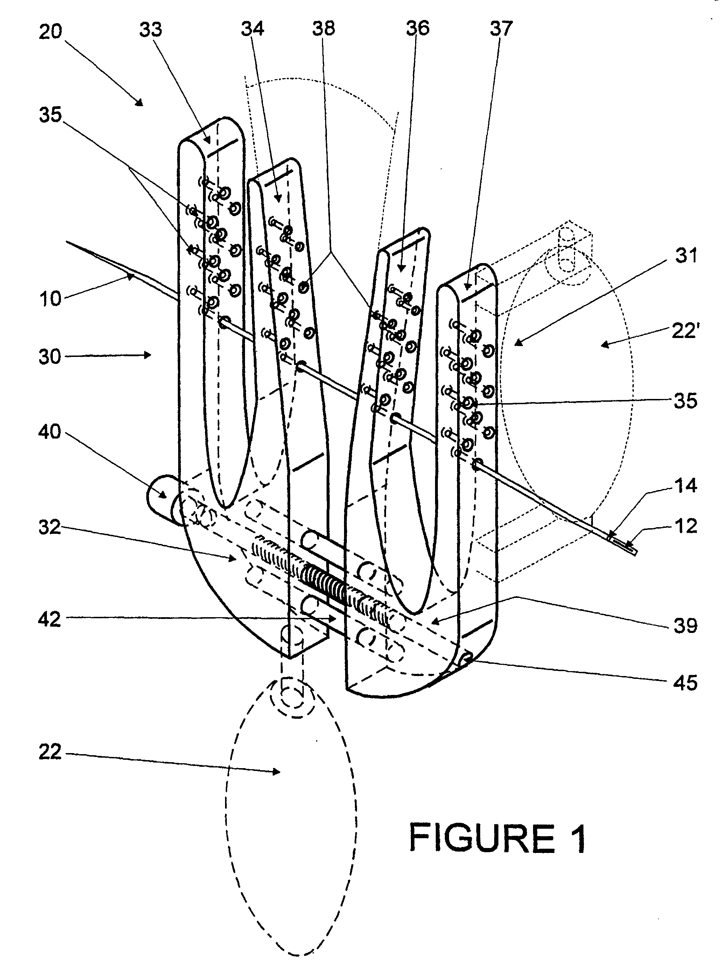

Sur la figure 1 est illustré un dispositif de rattrapage d'un tendon d'Achille comprenant d'une part un instrument 20 prévu pour être inséré en partie à l'intérieur de la gaine péritendineuse et d'autre part une aiguille 10 prévue pour passer un fil au travers du tendon supérieur remonté.

Comme illustré, l'instrument 20 se présente sous la forme de deux pièces en U 30, 31 assemblées par deux axes de maintien 42 et retenues avec un écartement voulu par une vis de réglage 40. Comme on peut l'observer, les deux pièces en U 30, 31 sont rigoureusement symétriques entre elles par rapport à un plan médian orthogonal à l'aiguille 10.

Chaque pièce en U présente donc deux branches 33/34, 36/37 réunies par une base de jonction 32/39. Les deux branches externes 33, 37 sont sensiblement rectilignes et assez épaisses, c'est-à-dire qu'elles présentent une hauteur de l'ordre de 70mm pour une section constante d'environ 7mm de large pour 12mm de long. A l'inverse, les deux branches internes 34, 36, sensiblement de même hauteur, présentent une section plus fine allant notamment en se rétrécissant vers le haut. Par exemple, les branches 34, 36 présentent vers le bas une section sensiblement rectangulaire de largeur de 5mm sur une longueur de 12mm, cette largeur étant réduite à environ 2mm vers le haut. Il convient de noter que les branches internes 34, 36 ne sont pas parallèles entre elles, comme les branches externes 33, 37, mais forment entre elles un angle ouvert vers le haut d'angle au sommet compris entre 2º et 8º, de préférence 4º. La longueur des branches 34/36 correspond à la hauteur de remontée maximum constatée du tendon par la contraction du muscle.

Les jonctions 32/39 présentent un volume suffisant pour contenir un mécanisme de liaison et de réglage des deux pièces en U 30, 31 l'une par rapport à l'autre. Plus particulièrement, ce mécanisme comprend d'abord une paire d'axes de maintien 42 solidaires de l'une des pièces, par exemple de la pièce 30, et coulissant dans des orifices borgnes en correspondance ménagés dans la jonction 39 de l'autre pièces 31. Ces deux axes 42 maintiennent donc les pièces 30 et 31 dans un même plan. Ce mécanisme comprend de plus une vis de réglage 40 montée mobile en rotation mais retenue axialement dans l'une des jonctions, par exemple celle 32 de la pièce 30, la tige filetée de cette vis 40 étant engagée dans un orifice taraudé 45 ménagé en correspondance dans l'autre jonction 39. Pour des considérations d'équilibre, cette vis de réglage est installée de préférence à mi-hauteur entre les deux axes 42.

Comme pour tout instrument de chirurgie, tous les bords des pièces 30; 31 sont arrondis pour éviter d'agripper, voire de couper, les tissus mous. Plus particulièrement, les extrémités supérieures des branches internes 34/36 sont arrondies selon deux courbes circulaires successives, une première courbe interne de rayon de l'ordre de 5mm, et une courbe d'extrémité externe de rayon de l'ordre de 1,7mm.

Selon l'invention, toutes les branches des deux pièces présentent un même réseau d'orifices 35, 38, quatre orifices respectivement de chaque branche étant toujours situés sur un même alignement de telle sorte à recevoir l'aiguille 10 tel qu'illustré sur la figure 1. Ces orifices présentent un diamètre interne compris entre 0,8mm et 2mm, de préférence 1,6mm, Ces orifices présentent des chanfreins d'entrée et de sortie de diamètres de l'ordre de 2,5mm. Compte tenu de la largeur de l'ordre de 7mm des branches externes 33 et 37, les orifices 35 de ces branches agissent en tant qu'orifices de guidage pour une aiguille 10 faisant que celle-ci est forcément conduite de telle sorte à pouvoir être insérée dans les orifices de passage 38 des branches internes 34, 36. Les chanfreins ménagés dans les embouchures de ces orifices assistent à cette insertion.

Si désiré, et en complément de la fonction de poignée des branches externes 33/37, on peut prévoir de compléter l'instrument 20 par des poignées plus importantes, donc de meilleure préhension. Par exemple, une première poignée 22 peut être agencée sous la jonction 32 de la pièce 30 dans son prolongement inférieur. En alternative, une poignée 22' peut être agencée en parallèle de l'une des branches externes 33 ou 37.

Comme la majorité des instruments utilisés en chirurgie, l'instrument 20 selon l'invention peut être réalisé en acier inoxydable poli pouvant être stérilisé après chaque opération. En alternative et pour des considérations de coût, l'instrument 20 peut également être réalisé en matière plastique stérile, cet instrument n'étant alors prévu que pour un usage unique.

En association avec cet instrument 20, le dispositif comprend une aiguille 10 de diamètre compris entre 0,7mm et 1,9mm, de préférence 1,5mm, qui est alors suffisamment rigide sans être traumatisante. La longueur de l'aiguille est de préférence supérieure à la largeur de l'instrument, donc de l'ordre de 80mm, bien que des aiguilles plus longues ou plus courtes soient également utilisables. Plus particulièrement, le chas 12 de cette aiguille est ménagé au sein d'une restriction 14 d'épaisseur moindre de telle sorte qu'avec le fil effectuant à ce niveau une boucle, le diamètre total reste inférieur à celui de 1,6mm des orifices 35, 38 par lesquels il est prévu de passer.

Grâce à ce dispositif, la technique opératoire de réparation du tendon d'Achille est sensiblement facilitée comme il va maintenant être expliqué ci-dessous. Après installation du patient puis anesthésie, le chirurgien pratique une petite incision, avantageusement verticale, de longueur réduite de l'ordre de 2-3cm. Le chirurgien peut alors introduire les branches internes 34 et 36 au travers de cette incision dans la gaine péritendineuse et faire remonter ces branches à l'intérieur de la gaine jusqu'à atteindre l'extrémité distale du tendon. Les branches 34, 36 présentant un angle ouvert, elles viennent naturellement se positionner de part et d'autre de ce tendon en épousant sa forme conique. La présence du tendon entre les branches 34, 36 peut être facilement constatée par palpation.

Dans cette situation, il suffit d'introduire l'aiguille 10 dans l'un des orifices de guidage 35 pour que, après pénétration au travers de la peau, elle passe forcément par l'orifice correspondant de la branche interne adjacente, par exemple 36, la conduisant alors directement dans le tendon qui, lors de son percement, est retenu par l'autre branche interne opposée 34. L'avance de l'aiguille 10 se poursuit en pénétrant dans l'orifice de passage de la branche 34 pour ressortir de la peau en passant par l'orifice de guidage 35 de la branche externe opposée 33. Deux autres fils complémentaires peuvent alors être enfilés rapidement de la même manière. Une fois le nombre désiré de fils passés, on tire lentement l'instrument 20 en arrière faisant que les branches internes 34, 36 se retirent de la gaine en emmenant avec elles les fils traversant le tendon. Ces six extrémités de fils sortent alors de la petite incision pratiquée au départ.

Cette opération de rattrapage est répétée sur l'autre extrémité du tendon, en l'occurrence celle rattachée au calcanéum. On obtient alors douze extrémités de fils qui permettent de ramener les extrémités de tendon bout-à-bout pour suture. Les fils sont également noués entre eux, les extrémités d'une boucle supérieure étant appariées avec celle d'une boucle inférieure.

Comme on a pu le constater à la lecture de cet exposé, ce simple dispositif facilite grandement une opération délicate à effectuer.

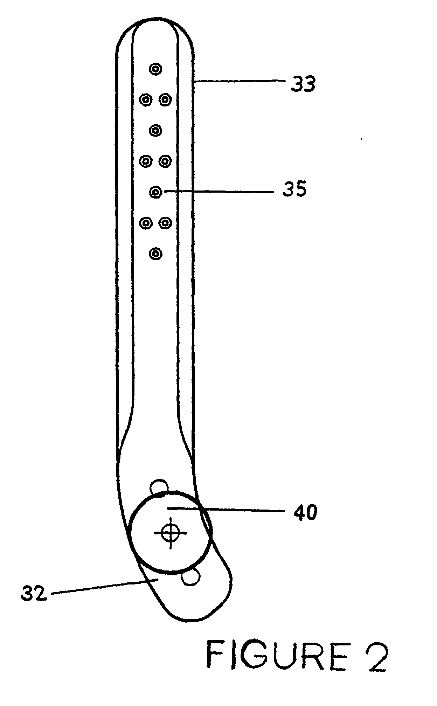

De nombreuses améliorations peuvent être apportées à l'instrument dans le cadre de l'invention. Notamment, et comme mieux visible sur la figure 2, les jonctions 32, 39 peuvent présenter une courbure permettant d'introduire les branches 34, 36 tout le long de la gaine péritendineuse sans risquer de venir frotter, voire heurter, le talon du pied.

Si désiré, on peut supprimer l'un des axes de maintien 42 pour n'en conserver qu'un seul, plus gros, travaillant en association avec la vis de réglage. Cet axe de maintien peut également ne pas présenter une section circulaire, mais une section polygonale, notamment circulaire ou rectangulaire empêchant une rotation des pièces en U l'une par rapport à l'autre. Par ailleurs on peut envisager un dispositif de blocage de la vis une fois l'écartement réglé, par exemple un contre-écrou agencé entre les deux pièces 30, 31.

## Revendications

1. Dispositif de rattrapage d'un tendon situé dans sa gaine péritendineuse **caractérisé en ce qu'**il comprend un instrument (20) composé d'une paire de branches internes (34,36) prévues pour être insérées dans la gaine de part et d'autre du tendon et d'une poignée externe (22,33,37) de manipulation dont un élément (33,37) est situé dans le plan de la paire de branches internes(34,36) et en vis-à-vis, l'élément et la paire de branches internes présentant respectivement au moins un orifice (35,38) selon un même alignement passant par le tendon ; et une aiguille (10) susceptible d'être insérée dans l'orifice (35) (38) de l'élément pour être guidée afin de passer par les orifices de branches internes.

2. Dispositif selon la revendication 1, **caractérisé en ce que** l'élément de poignée (33,37) et la paire de branches internes (34,36) présentent respectivement un même réseau d'orifices (35,38) alignés, si désiré, les embouchures des orifices des branches étant chanfreinées.

3. Dispositif selon la revendication 1 ou 2, **caractérisé en ce que** les deux branches internes (34,36) forment entre elles un angle d'angle au sommet compris entre 2° et 8°, de préférence 4°, ouvert dans le sens d'introduction.

4. Dispositif l'une des revendications 1 à 3, **caractérisé en ce que** les branches internes (34,36) de l'instrument (20) présentent une section transversale rectangulaire aplatie aux coins arrondis, ou une section ovale aplatie, si désiré la plus grande longueur de cette section se rétrécissant vers l'extrémité.

5. Dispositif selon l'une des revendications 1 à 4, **caractérisé en ce que** l'écartement entre les deux branches internes (34,36) est réglable.

6. Dispositif selon l'une des revendications 1 à 5, **caractérisé en ce que** l'élément de la poignée comprend deux branches externes (33,37) respectivement situées de part et d'autre de la paire de branches internes (34,36) dans un même plan.

7. Dispositif selon les revendications 5 et 6, **caractérisé en ce que** l'instrument (20) est constitué de deux pièces sensiblement en U (30,31) et assemblées côte-à-côte par un mécanisme (40,42) permettant de régler l'écartement entre les pièces, les branches de U adjacentes de chacune des pièces formant la paire de branches internes, les branches éloignées formant la poignée.

8. Dispositif selon la revendication 7, **caractérisé en ce que** les deux pièces en U (30,31) sont symétriques par rapport à un plan orthogonal, et, si désiré, **en ce que** la base de jonction (32,39) des branches des pièces en U est arquée.

9. Dispositif selon la revendication 7 ou 8, **caractérisé en ce que** le mécanisme d'assemblage des deux pièces côte-à-côte comprend au moins une tige (42) solidaire de l'une (30) des pièces et coulissant dans un perçage ménagé en correspondance dans l'autre pièce (31) ainsi qu'une vis (40) montée mobile en rotation dans l'une (30) des pièces avec son filetage engagé dans un trou taraudé (45) ménagé dans l'autre pièce(31), la tête de la vis étant apparente à l'extérieur pour manipulation.

10. Dispositif selon l'une des revendications précédentes **caractérisé en ce que** le chas (12) de l'aiguille (10) présente une épaisseur réduite.

11. Dispositif selon l'une des revendications précédentes, **caractérisé en ce que** l'instrument et l'aiguille sont réalisés en acier inoxydable, ou **en ce que** l'instrument à usage unique est réalisé en plastique.

## Patentansprüche

1. Vorrichtung zum Ergreifen einer Sehne in ihrem umhüllenden Gewebe,
**dadurch gekennzeichnet, daß** ein Instrument (20) vorgesehen ist, das zusammen gesetzt ist aus einem Paar innerer Zweige (34, 36), die vorgesehen sind, in die Hülle von einer Seite und der anderen der Sehne eingeführt zu werden, und einem äußeren Griff (22, 33, 37) zur Handhabung, von dem ein Element (33, 37) angeordnet ist in der Ebene des Paars der inneren Zweige (34, 36) und gegenüber weisen das Element und das Paar der inneren Zweige jeweils mindestens eine Öffnung (35, 38) auf entlang einer einzigen Ausrichtung durch die Sehne und einer Nadel (10), die dazu vorgesehen ist in die Öffnung (35) (38) des Elements eingeführt zu werden, um durch die Öffnungen der inneren Zweige geführt zu werden.

2. Vorrichtung gemäß dem Anspruch 1, **dadurch gekennzeichnet, dass** das Element zum Greifen (33, 37) und das Paar der inneren Zweige (34, 36) jeweils ein selbes Netz von ausgerichteten Öffnungen (35, 38) aufweisen, wobei vorzugsweise die Mündungen der Öffnungen der Zweige angeschrägt sind.

3. Vorrichtung gemäß dem Anspruch 1 oder dem Anspruch 2, **dadurch gekennzeichnet, dass** die beiden inneren Zweige (34, 36) zwischen sich einen Winkel bilden, wobei der Winkel am oberen Ende zwischen 2° und 8°, vorzugsweise 4°, und offen in Richtung des Zugangs ist.

4. Vorrichtung gemäß einem der Ansprüche 1 bis 3, **dadurch gekennzeichnet, dass** die inneren Zweige (34, 36) des Instruments (20) einen transversalen, rechteckigen Abschnitt bilden, der abgeplattet ist an den gerundeten Ecken oder einen oval abgeplatteten Abschnitt, wobei sich vorzugsweise die größte Länge dieses Abschnitts nach außen hin verkürzt.

5. Vorrichtung gemäß einem der Ansprüche 1 bis 4, **dadurch gekennzeichnet, dass** der Abstand zwischen den inneren Zweigen (34, 36) einstellbar ist.

6. Vorrichtung gemäß einem der Ansprüche 1 bis 5, **dadurch gekennzeichnet, dass** der Griff zwei äussere Zweige (33, 37) aufweist, die jeweils beidseits des Paars der inneren Zweige (34, 36) in einer Ebene angeordnet sind.

7. Vorrichtung gemäß den Ansprüchen 5 und 6, **dadurch gekennzeichnet, dass** das Instrument (20) aus zwei im wesentlichen U-förmigen Teilen (30, 31) gebildet und Seite an Seite mittels einer Mechanik zusammen gesetzt ist, die es ermöglicht den Abstand zwischen den Teilen einzustellen, wobei die aneinander liegenden Zweige des Us von jedem der Teile das Paar der inneren Zweige bilden und die beabstandeten Zweige den Griff bilden.

8. Vorrichtung gemäß dem Anspruch 7, **dadurch gekennzeichnet, dass** die zwei U-förmigen Teile (30, 31) symmetrisch sind hinsichtlich einer rechtwinkligen Ebene und vorzugsweise dadurch, dass die Basis der Verbindung (32, 39) der Zweige der U-förmigen Teile gewölbt ist.

9. Vorrichtung gemäß dem Anspruch 7 oder 8, **dadurch gekennzeichnet, dass** die Mechanik zum Zusammensetzen der beiden Teile neben einander mindestens einen Schaft (42) aufweist, der einteilig ist mit einem (30) der Teile und gleitend ist, in einer Bohrung, die entsprechend in dem anderen Teil (31) untergebracht ist, sowie eine Schraube (40), die drehbar in einem (30) der Teile montiert ist und mit ihrem Gewinde in ein gebohrtes Loch (45) eingreift, das in dem anderen Teil (31) enthalten ist, wobei der Kopf der Schraube zur Handhabung nach außen sichtbar ist.

10. Vorrichtung gemäß einem der vorhergehenden Ansprüche, **dadurch gekennzeichnet, dass** das Nadelöhr (12) der Nadel (10) eine reduzierte Dicke aufweist.

11. Vorrichtung gemäß einem der vorhergehenden Ansprüche, **dadurch gekennzeichnet, dass** das Instrument und die Nadel mit nicht oxidierendem Stahl ausgeführt sind oder dadurch, dass das Einweg-Instrument in Plastik ausgeführt ist.

## Claims

1. Device for gripping a tendon situated in its peritendon, **characterised in that** it comprises an instrument (20) composed of a pair of internal arms (34, 36) designed to be inserted in the sheath on each side of the tendon and an external manipulation handle (22, 33, 37), one element (33, 37) of which is situated in the same plane as the pair of internal arms (34, 36) and facing them, the element and the pair of internal arms having respectively at least one orifice (35, 38) in the same alignment passing through the tendon; and a needle (10) able to be inserted in the orifice (35) (38) in the element in order to be guided so as to pass through the orifices in the internal arms.

2. Device according to Claim 1, **characterised in that** the handle element (33, 37) and the pair of internal arms (34, 36) have respectively the same network of aligned orifices (35, 38), if desired, the openings of the orifices in the arms being bevelled.

3. Device according to Claim 1 or 2, **characterised in that** the two internal arms (34, 36) form between them an apex angle of between 2° and 8°, preferably 4°, open in the direction of insertion.

4. Device according to one of Claims 1 to 3, **characterised in that** the internal arms (34, 36) of the instrument (20) have a flattened rectangular transverse section with rounded corners, or a flattened oval section, the greatest length of this section narrowing towards the end if required.

5. Device according to one of Claims 1 to 4, **characterised in that** the separation between the two internal arms (34, 36) is adjustable.

6. Device according to one of Claims 1 to 5, **characterised in that** the handle element comprises two external arms (33, 37) respectively situated on each side of the pair of internal arms (34, 36) in the same plane.

7. Device according to Claims 5 and 6, **characterised in that** the instrument (20) consists of two substantially U-shaped pieces (30, 31) assembled side by side by a mechanism (40, 42) for adjusting the separation between the pieces, the adjacent arms of the U of each of the pieces forming the pair of internal arms, the distant arms forming the handle.

8. Device according to Claim 7, **characterised in that** the two U-shaped pieces (30, 31) are symmetrical with respect to an orthogonal plane and, if required, **in that** the base (32, 39) where the arms of the U-shaped pieces join is curved.

9. Device according to Claim 7 or 8, **characterised in that** the mechanism for assembling the two pieces side by side comprises at least one rod (42) fixed to one (30) of the pieces sliding in a drilling provided correspondingly in the other piece (31) and a screw (40) mounted so as to be able to move rotationally in one (30) of the pieces with its thread engaged in a threaded hole (45) provided in the other piece (31), the head of the screw being visible outside for manipulation.

10. Device according to one of the preceding claims, **characterised in that** the eye (12) of the needle (10) has a reduced thickness.

11. Device according to one of the preceding claims, **characterised in that** the instrument and the needle are produced from stainless steel, or **in that** the single-usage instrument is produced from plastics material.
